## Europäisches Patentamt
## European Patent Office
## Office européen des brevets

(19)

(11) Numéro de publication: **0 126 009**
**B1**

# FASCICULE DE BREVET EUROPÉEN

(12)

(45) Date de publication du fascicule du brevet:
14.01.87

(21) Numéro de dépôt: **84400999.3**

(22) Date de dépôt: **16.05.84**

(51) Int. Cl.⁴: **C 07 K 7/04**, C 07 K 5/04,
C 07 K 1/00, A 61 K 37/64,
A 61 K 37/02

(54) Nouveaux dérivés de peptides, leur préparation et leur application comme inhibiteurs de l'élastase.

(30) Priorité: 16.05.83 FR 8308052

(43) Date de publication de la demande:
21.11.84 Bulletin 84/47

(45) Mention de la délivrance du brevet:
14.01.87 Bulletin 87/3

(84) Etats contractants désignés:
AT BE CH DE FR GB IT LI LU NL SE

(56) Documents cité:
EP-A-0 000 330
CH-A-620 671
FR-A-2 183 170
FR-A-2 293 439
FR-A-2 411 174
FR-M-2 907
FR-M-2 957
NL-A-279 057

UNLISTED DRUGS, vol. 19, no. 6, 1967, page 64a, no.
R1, Spec. Library Associat.;

(73) Titulaire: **Etablissement Public dit: CENTRE
NATIONAL DE LA RECHERCHE SCIENTIFIQUE
(CNRS), 15, Quai Anatole France, F-75700 Paris
(FR)**

(72) Inventeur: **Robert, Ladislas, 7, rue Jean- Baptiste
Lully, F-94440 Santeny (FR)**
Inventeur: **Hornebeck, William, 5, rue Jean- Jaurès,
F-78210 Saint Cyr L'Ecole (FR)**
Inventeur: **Moczar, Elemer, 14, Allée de la
Gambaudrie, F-91190 Gif Sur Yvette (FR)**

(74) Mandataire: **Nony, Michel, Cabinet Nony 29, rue
Cambacérès, F-75008 Paris (FR)**

**0 126 009**

## Description

On sait que l'élastine est une protéine fibreuse élastique du tissu conjonctif des vertébrés. Elle est présente dans les parois vasculaires, la peau, les poumons, les cartilages, les ligaments et d'autres tissus. L'élastine est la protéine la plus résistante de l'organisme. Par contre, sa dégradition augmente particulièrement vite dans certains états pathologiques et en général au cours du vieillissement, dans tous les tissus riches en élastine comme les parois vasculaires et le derme; voir L. ROBERT, in "Précis de physiologie cutanée", sous la direction de J. MEYNADIER, Ed. de la Porte Verte (1980) p. 155-173.

Seules quelques protéases peuvent attaquer l'élastine. Ces protéases sont appelées des élastases ou des protéases de type élastase. De telles enzymes sont l'élastase pancréatique et les élastases cellulaires: élastases leucocytaire et plaquettaire, et élastases de macrophages, de fibroblastes et de cellules musculaires lisses artérielles.

Ces enzymes sont capables de dégrader l'élastine dans les tissus et organes mentionnés ci-dessus, et de contribuer au développement de maladies telles que l'artériosclérose, l'emphysème, l'arthrose, le diabéte, et aussi au vieillissement des tissus conjonctifs de l'organisme.

L'activité des élastases est contrôlée et régulée par des inhibiteurs naturels qui sont présents d'une part das le plasma sanguin, comme l'alpha 1-antitrypsine et l'alpha 2-macroglobuline et d'autre part dans les sécrétions tissulaires telles que les sécrétions bronchiques; voir par exemple HORNEBECK et al: "Control of elastic tissue destruction by elastase inhibitors" in DEYL, ADAM Eds, Connective Tissue Research: Chemistry, Biology and Physiology, p. 233-246, A.R. Liss. Inc., New York 1981.

En outre, de nombreuses bactéries capables de pénétrer dans l'organisme sécrètent également des protéases élastolytiques dont l'action contribue d'une façon substantielle à leur effet pathogène.

On sait également que la progression des tumeurs malignes (cancers, sarcomes) dans l'organisme et la formation de métastases, souvent fatales pour le malade, sont aussi conditionnées par la sécrécion de protéases du type élastage; voir par exemple Biological significance of Elastase-like enzymes in Arteriosclerosis and Human breast cancer. HORNEBECK W., BRECHEMIER D., BELLON C., ADNET J.J. and ROBERT L. in: P. Straülli, A.J. Barrett, A. Baici eds. Proteinases and tumor invasion. vol 6, of ORTC Monograph series (1980) pp. 117-141, (Raven Press, New York). De telles enzymes sont capibles de détruire les tissus environnants et de rendre ainsi possible la pénétrition des cellules malignes dans la circulation sanguine et de provoquer l'invasion de l'organisme par la tumeur.

Pour toutes ces raisons, il est important de pouvoir disposer d'inhibiteurs capables de contrôler l'activité des élastases.

Toutefois certaines de ces élastases ont une activité utile, voire indispensable, pour l'organisme, par exemple dans la digestion des bactéries phagocytées par les macrophages.

Il apparaît donc important de disposer à la fois d'inhibiteurs d'élastases et de protecteurs des fibres élastiques. Il apparaît encore préférable de pouvoir disposer d'inhibiteurs d'élastases qui sont capables d'agir sélectivement au niveau des fibres élastiques dont l'intégrité est indispensable pour le bon fonctionnement de l'organisme.

En effet, l'hydrolyse enzymatique de l'élastine par les élastases peut être considérée comme un facteur déterminant dans de nombreuses pathologies des tissus élastiques: irtériosclérose, emphysème et certaines maladies de la peau. Dans l'organisme vivant cette protéolyse résulte d'un déséquilibre entre le taux des protéases possédant une activité élastolytique d'une part et d'autre part le taux d'inhibiteurs naturels d'origine plasmatique ou tissulaire. L'une des approches thérapeutiques qui a été envisagée dans le cas d'une déficience d'origine génétique ou fonctionnelle de ces inhibiteurs de protéases, consiste en l'utilisation d'inhibiteurs de substitution naturels (alpha 1-antitrypsine).

Toutefois l'utilisation d'inhibiteurs naturels présente de nombreux inconvénients parmi lesquels le coût du traitement et le risque d'accidents d'origine immunologique. D'autre part les inhibiteurs des élastases utilisés en thérapeutique animale expérimentale dans le cas de l'emphysème ont l'inconvénient de posséder une forte toxicité.

La présente invention a pour objet de nouveaux lipopeptides synthétiques bifonctionnels qui peuvent être considérés à la fois comme des inhibiteurs de l'activité élastolytique et des protecteurs de la fibre élastique. Ces substances sont en effet capables de reconnaître la fibre élastique et de se fixer sur elle et d'autre part de reconnaître et de neutraliser le site actif des élastases.

Les inhibiteurs d'élastases de l'invention présentent notamment les avantages suivants: ils ne présentent pas de caractère antigénique; ils sont biodégradables; ils ont en outre la propriété de parvenir au site de leur action et de s'y fixer.

La présente invention a donc pour objet de nouveaux lipopeptides de formule générale:

$$R-X-(P_1)_x-(L-Ala-L-Ala-P_2)-A \quad (I)$$

dans laquelle:

x représente le nombre 0 ou 1;

R représente le reste acyle d'un acide carboxylique hydrophobe tel qu'un acide carboxylique aliphatique ayant 6 à 25 atomes de carbone et pouvant renfermer éventuellement 1 à 5 doubles liaisons, d'un acide carboxylique alicyclique ayant 6 à 25 atomes de carbone, d'un acide arylcarboxylique ou d'un acide carboxylique arylaliphatique dont le groupement aryle comprend 1 à 2 cycles et dont le groupement aliphitique comprend 1 à 18 atomes de carbone, les groupements aromatiques desdits groupements aryle ou

2

arylaliphatique étant éventuellement substitués;

$P_2$ représente un résidu d'acide aminé ou de dipeptide, relié par son extrémité N-terminale au groupement L-Ala adjacent, choisi dans le groupe constitué par les acides aminés et dipeptides suivants:

-L-Ala-, -L-Val-, -Gly-, -L-Mét-, -L-Pro-, -L-Leu-, -L-Pro-L-Val-, -L-Pro-L-Ala-, -L-Pro-L-Phé-, -L-Pro-L-Leu, -L-Pro-L-Mét- et -L-Pro-Gly-;

$P_1$ représente un résidu d'acide aminé ou d'oligopeptide formé de 2 à 8 aminoacides de la série L, lesdits aminoacides étant choisis dans le groupe constitué par la glycine, l'alanine, la valine, la méthionine, la leucine, l'isoleucine, la proline, la phénylalanine, la sérine, la cystéine, la cystine, l'arginine, la tyrosine, l'ornithine, la lysine et l'acide glutamique,

étant entendu que $P_1$ est relié à RX- par son groupement N-terminal et que $P_2$ est relié à A par son groupement C-terminal,

Ala est la représentation conventionnelle de l'alanine,

X représente une liaison covalente directe reliant R soit au groupement N-terminal (-NH-) du premier aminoacide de $P_1$ (cas où x = 1) soit au groupement N-terminal (-NH-) du premier groupement Ala représenté à gauche sur la formule I (cas où x = 0),

ou bien X est un groupement divalent ayant 2 à 10 atomes de carbone jouant le rôle de "bras" entre le groupement R et le reste de la molécule de formule I,

et A représente la partie C-terminale du peptide $-(P_1)_x$-(Ala-Ali-$P_2$) -A, A étant choisi parmi un groupement carboxylique -$CO_2H$ ou ses dérivés, -CHO, -$CONH_2$, -$COCH_2Cl$ et -$CH_2OH$.

Parmi les dérivés de formule 1 on citera notamment ceux pour lesquels R représente le reste acyle d'un acide gras ayant 6 à 20 atomes de carbone tel que l'acide laurique ou l'acide oléique ou le reste d'un autre acide organique à caractère hydrophobe tel que le reste de l'acide chénodéoxycholique, de l'acide cholique, etc...; ou bien R- représente par exemple le reste acyle d'un acide phénylalcanoïque éventuellement substitué sur le noyau benzénique (par exemple par des groupements halogène, triflourméthyle, hydroxyle ou alkyle inférieur ayant 1 à 3 atomes de carbone); lorsque X est un groupement divalent jouant le rôle de bras il s'agit par exemple d'un groupement -Z-$(CH_2)_n$-CO-, Z représentant -O- ou -NH-, et n'étant un nombre entier variant de 5 à 20; le bras X peut être substitué par un ou plusieurs groupements tels que -OH, -$NH_2$, -COOH, de façon à contribuer à la solubilisation ou à permettre la dérivatisation éventuelle du composé I; de préférence l'oligopeptide que représente $-(P_1)_x$-(L-Ala-L-Ala-$P_2$) - ne possède pas plus de 10 aminoacides; étant entendu que le groupement C-terminal que représente A peut être non seulement un groupement carboxylique ou un de ses dérivés mais encore un des groupements indiqués ci-dessus.

Parmi les dérivés carboxylique de la fonction que peut représenter A, on citera notamment les esters, en particulier les esters de formule -CO-OY,Y étant un groupement aliphatique, aryle ou arylaliphatique éventuellement substitué

Y est en particulier un groupement alkyle ayant 1 à 5 atomes de carbone ou un groupement phényle du phénylalkyle, éventuellement substitué.

Parmi les dérivés du groupement carboxylique que peut représenter A, on citera également les sels métalliques, notamment les sels des métaux alcalins ou alcalino-terreux (en particulier sodium, potassium, calcium, etc...), les sels d'ammonium, et les sels formés avec des produits aminés tels que par exemple l'éthanolamine, la lysine, l'arginine, les bétaïnes, la pyridoxine (en tant que cofacteur de la lysyl-oxydase intervenant dans la synthèse de l'élastine), et d'autres molécules basiques, y compris des vitamines.

L'invention a également pour objet un procédé de préparation des composés de formule I, selon les méthodes connues de préparation des peptides et de leurs dérivés.

L'invention a en particulier pour objet un procédé de préparation des composés de formule 1, caractérisé par le fait que l'on utilise comme produit de départ un composé de formule générale II

H-$X_1$-(-$P_1$-)-(L-Ala-L-Ala-$P_3$-)-$A_1$ (II)

dans laquelle $X_1$ a la même définition que X ou bien représente une liaison covalente directe entre H- et -$P_1$-;

$P_3$ a la même définition que $P_2$, ou $P_3$ représente un groupement -L-Pro-, ou bien $P_3$ représente une liaison covalente directe entre $A_1$ et le groupement -L-Ala- immédiatement adjacent;

$A_1$ représente un groupement - $CO_2H$, - CO-OY (Y étant défini comme précédemment), - CHO ou - $CONH_2$;

et x est défini comme précédemment;

que l'on fait réagir ledit produit de départ, éventuellement présent sous la forme d'un sel d'addition comme le chlorhydrate, avec un réadtif de

R-$X_2$-$Z_1$ (III)

dans laquelle R est défini comme précédemment

$X_2$ a la même définition que X lorsque $X_1$ représente une liaison convalente, et $X_2$ représénte une liaison convalente directe entre R et $Z_1$ lorsque $X_1$ a la même définition que X; et $Z_1$ est un groupement réactif permettant la réaction de R-$X_2$-$Z_1$ sur le composé II avec éliminition d'un composé $Z_1H$ et formition d'un composé de formule IV

R-X-$(P_1)_x$-(L-Ala-L-Ala-$P_3$-)$_y$-$A_1$ (IV);

que, dans le cas où $P_3$ représente le groupement -L-Pro-, on fait réagir le composé de formule IV avec un acide aminé de la série L choisi parmi la valine, l'alanine, la phénylalaline, la leucine, la méthionine et la glycine, ou avec un dérivé d'un desdits acides aminés, (en particulier un dérivé dont le groupement C-terminal est un groupement A tel que défini précédemment), pour obtenir un dérivé de formule I; puis que l'on transforme, si désiré, le composé obtenu en tout autre composé de formule I selon les méthodes connues, en

3

particulier en remplaçant le groupement terminal A ou A$_1$ en tout autre groupement terminal répondant à la définition de A donnée ci-dessus.

Dans des modes d'exécution préférés, le procédé d'invention peut encore présenter les caractéristiques suivantes prises isolément ou en combinaison:

- Z$_1$ est par exemple un halogène tel que le chlore ou le brome;

- pour préparer un composé de formule I pour lequel A représente -CH$_2$OH, on peut notamment faire rèagir le composé IV (avec P$_3$=L-Pro) avec un dérivé d'acide aminé pour lequel le groupement C-terminal est -CH$_2$OH-; on opère par exemple en faisant réagir ledit dérivé (tel que le vilinol) en présence de N-méthyl morpholine et de chlorure de t-butyl carbonyle; voir notamment ANDERSON C.W. et al, J. Am. Chem. Soc., 89, 5012 (1967).

- pour préparer un composè de formule I pour lequel A représente -CHO, on peut par exemple soumettre un composé de formule I pour lequel A représente -CH$_2$OH à l'action d'un agent d'oxidation tel que par exemple le diméthylsulfoxyde (PFITZNER et al, J. Am. Chem. Soc, 87, 7661 (1965) en présence d'un catalyseur approprié comme l'acide phosphorique ou dichloroacétique; THOMPSON C.R., Biochemestry, 12, 47 (1973);

- pour préparer un composé de formule I dans laquelle A représente -COCH$_2$Cl, on peut par exemple faire réagir le composé IV (P$_3$=L-Pro) avec un dérivé d'acide aminé dont le groupement C-terminal est -CO-CH$_2$Cl; voir par exemple THOMPSON et al C.R., Biochemistry, 12, 44 (1973).

- pour préparer un composé de formule I pour lequel A représente le groupement ester -CO-OY, on fait réagir par exemple le composé de formule I (A=-CO$_2$H) avec l'alcool choisi en présence d'un agent déshydratant tel que le chlorure de thionyle;

- pour préparer un composé de formule I pour lequel A représente -CONH$_2$, on peut par exemple faire réagir le composé de formule IV (P$_3$= L-Pro) avec un acide aminé dont le groupement C-terminal est -CONH$_2$, par la méthode des anhydrides mixtes; Thompson et al, Biochemistry, 12, 57 (1973).

Les composés de formule I présentent des propriétés intéressantes. Ils possèdent notamment la double propriété d'inhiber l'activité des protéases du type élastase et de se fixer sur la fibre d'élastine.

En outre, ils n'ont pas de toxicité appréciable aux doses actives. Toutefois les composés de formule I pour lesquels A représente -CO-CH$_2$Cl ont une certaine toxicité, généralement à des doses supérieures à 20 mg/Kg, mais ces composés ont une activité inhibitrice des élastases très élevée, de sorte que leur index thérapeutique n'est pas moins favorable que pour les autres composés de formule I.

La présente invention a aussi pour objet l'utilisation des composés de formule I comme inhibiteurs des élastases et/ou protecteurs de la fibre élastique, notamment dans des compositions comprenant un composé de formule I avec un excipient approprié.

Les composés de formule I sont utilisables par exemple comme médiciments à titre de traitement principal ou de traitement complémentaire dans les cas d'artériosclérose, d'emphysème pulmonaire, d'arthrose, de diabète et de certaines tumeurs dans lesquelles les élastases peuvent être impliquées.

Les compositions de l'invention sont notamment des compositions pharmaceutiques caractérisées par le fait qu'elles comprennent à titre d'ingrédient actif au moins un composé de formule I, éventuellement en mélange avec un excipient approprié.

Ces compositions pharmaceutiques sont administrées par voie parentérale, rectale, topique ou orale, ou par inhalation d'aérosols.

A cet effet elles peuvent être présentées sous la forme de solutions aqueuses (solutions injectables ou buvables) ou solutions en conditionnement pressurisé pour aérosols d'émulsions, de préparations semi-solides (crèmes, suppositoires), ou sous forme de poudre lyophilisée à diluer ou contenue dans une capsule ou gélule ingérable.

Dans les compositions pharmaceutiques de l'invention (à l'exception des poudres lyophilisées, les composés de formule I sont présents généralement à une concentration de 0,1 à 5 % en poids.

La posologie dépend notamment en fonction de la voie d'idministrition et de l'effet thérapeutique recherché. Par exemple chez l'adulte elle peut varier de 50 mg à 5 g de principe actif par jour.

Les composés de formule I présentent également des propriétés intéressantes lorsqu'ils sont appliqués sur la peau, notamment des propriétés d'inhibition de l'élastolyse cutanée. En particulier ils permettent de conserver ou de restaurer la souplesse de la peau et de prévenir ou de retarder la formation de rides notamment sur la peau du visage, du cou et des mains (effet anti-vieillissement), et l'invention a aussi pour objet l'utilisation des composés de formule I dans ce but.

Les composés de formule I sont donc susceptibles d'améliorer l'aspect de la peau et la présente invention a également pour objet des compositions cosmétiques pour la peau, caractérisées par le fait qu'elles comprennent au moins un composé de formule I.

Elles comprennent en outre au moins un adjuvant ou excipient habituellement utilisé dans les compositions cosmétiques pour la peau.

Ces compositions cosmétiques pour la peau peuvent être présentées par exemple sous forme de crème, de gel, d'émulsion ou de solution aqueuse alcoolique ou hydroalcoolique.

La concentration du composé de formule I dans ces compositions pour la peau varie généralement de 0,1 à 2 % en poids.

Les adjuvants généralement présents dans ces compositions cosmétiques sont par exemple des parfums, des colorants, des agents conservateurs, des agents épaississants ou des agents émulsionnants.

Ces compositions pour la peau constituent notamment des crèmes, des laits ou des lotions pour le corps, les

0 126 009

mains ou le visage, y compris des crèmes, laits ou lotions anti-solaires.

L'invention a également pour objet un procédé de traitement cosmétique, caractérisé par le fait que l'on applique sur la peau au moins un composé de formule I à l'aide d'une composition cosmétique telle que définie ci-dessus.

Les exemples suivants illustrent l'invention sans toutefois la limiter:

**Exemple 1**

Préparation de l'oléoyl-L-Alanyl-L-Alanyl-L-Prolyl-L-Alanine

On dissout 3,65g (0,01M) de L-Alanyl-L-Alanyl-L-Propyl-L-Alanine (chlorhydrate) préparé par une méthode connue, dans un mélange de 60ml d'éthanol à 90% et de 3,35g de triéthylamine. On y ajoute sous agitation, à 4°C lg (0,025M) de chlorure d'oléoyle goutte à goutte pendant 15 minutes puis on agite encore 4 heures à la température ambiante.

On chasse l'éthanol sous vide, on ajoute au résidu 100ml d'eau. On ajuste le pH à 8.5 par l'addition de triéthylamine, on extrait l'excédent d'acide oléique par l'éther de pétrole. On ajuste le pH de la phase aqueuse à 4 par l'addition d'acide acétique. On extrait l'oléoyl peptide par l'acétite d'éthyle.

On chasse le solvant sous vide. On dissout le résidu dans le minimum d'acétate d'éthyle et on amorce la cristallisation par addition d'éther de pétrole.

F = 115-120°C; $\alpha_D$ = -76° (C = 0,5 %, éthanol).

**Exemple 2**

Préparation de lauroyl-trialanine

On dissout 1g de Tri-Alanine dans 15ml d'éthanol à 80% en présence de 0,6g (0,83ml) de triéthylamine. On y ajoute goutte à goutte sous agitation à la température ambiante 1,1g (1,19ml) de chlorure de lauroyle.

On abandonne le mélange 2 heures à la température ambiante. On y ajoute 5ml d'eau, on extrait le mélange par l'éther de pétrole, on ajoute à la phase inférieure 30ml d'eau, on laisse reposer 1 heure à la température ambiante. On filtre, on lave les cristaux par l'éther de pétrole et par l'eau.

On recristallise la substance dans l'alcool à 80%.

F:219°C $\alpha_D$ = -86° (C = 0,5 %, éthanol).

**Exemple 3**

Oléoyl-L-Alanyl-L-Alanyl-L-Prolyl-L-Alaninal.

3a) Méthyl ester d'Oléoyl-L-Alanyl-L-Alanyl-L-Proline.

On dissout 3,5 g d'acide oléique dans 1,2 ml de tétrahydrofuranne.

On y ajoute à -15°C 1,32 ml de N-méthyl morpholine et 1,50 ml de chlorure de t-butyl-carbonyle.

Le mélange est agité 10 minutes à cette température, puis on y ajoute 3,08g de chlorohydrate de méthyl ester de L-alanyl-L-alanyl-L-proline, dissout dans un mélange de 10 ml de diméthylformamide et de 1,2 ml de N-méthylmorpholine.

On laisse monter la température à 0°, om agite le mélange 1 heure à cette température, on laisse reposer la nuit à la température ambiante.

On essore les cristaux précipités de chlorohydrate de N-méthylmorpholine.

On chasse le solvant des eaux-mères sous vide et on cristallise la substance dans un mélange d'acétate d'éthyle-hexane.

$\alpha_D$ = - 76° (C = 0,5 % éthanol)

Rf: 0,5, couche de silice, méthanol-chloroforme 5:95 v/v.

3b) Oléoyl-L-Alanyl-L-Alanyl-L-Proline.

On dissout 1,07 g de méthyl ester d'oléoyl-L-alanyl-L-alanyl-Lproline dans 10 ml de méthanol. On y ajoute 3 ml d'une solution aqueuse de NaOH (1 M) et on abandonne la solution 3 heures à la température ambiante on ajuste le pH à 4 à l'aide de HCl, et on chasse le solvant sous vide.

On reprend le résidu dans l'acétate d'éthyle, et on cristallise le produit dans l'éther-acétate d'éthyle.

$\alpha_D$ = - 78° (C = 0,5 % éthanol).

3c) Oléoyl-Alanyl-Alanil-Prolyl-Alaninol.

On dissout 1,04g (2mM) d'oléoyl-Alanyl-Alanyl-Proline dans 20ml de diméthylformamide.

On y ajoute à -15°C 0,24ml (2,2mM) de N-méthyl morpholine et 0,29ml (2,2mM) de chlorure de t-butyl carbonyle. Le mélange est agité 10 minutes à cette température, puis on y ajoute 0,156ml de L-Alaninol. On laisse monter la température à 0°C et on agite le mélange 2 heures à cette température. On laisse reposer la nuit à la température ambiante. On chasse le solvant sous vide, on reprend le résidu dans l'acétate d'éthyle, on

5

lave la phase successivement par eau, par une solution aqueuse acide chlorhydrique à 5% et par une solution aqueuse de carbonate de sodium à 5%.

On chasse le solvant sous vide. On dissout le résidu dans le minimum d'acétate d'éthyle et on amorce la cristallisation par addition d'éther de pétrole (cristaux déliquescents).

$\alpha_D = -72°$ (C = 0,5%, éthanol).

3d) Oléoyl-Alanyl-Alanyl-Prolyl-Alaninal.

On dissout 2,9g (5mM) d'oléoyl-Alanyl-Alanyl-Prolyl-Alaninol dans 18ml de chloroforme. On y ajoute 2ml de diméthylsulfoxyde et 3,1g (15mM) de dicyclohexylcarbodiimide.

Ensuite on ajoute à la solution agitée à 0,86ml (15mM) d'acide phosphorique à 90% dans une heure, distribuée en 6 doses égales.

Après 4 heures d'agitation, on chasse le solvant sous vide. On reprend le résidu dans 30ml de chloroforme et on refroidit la solution à -30°C, on essore les cristaux séparément (dicyclohexylurée) et on lave les eaux-mères par l'eau. La substance peut être isolée à partir de la phase aqueuse par chromatographie préparative sur couches minces

$\alpha_D = -70°$ (C = 0,5 %, éthanol)

$R_f = 0,8$, couche de silice, chloroforme-méthanol = 15:1.


## Exemple 4

Préparation de l'oléoyl-tri alanine.

On dissout 2 g de L-alanyl-L-alanyl-L-alanine dans 30 ml d'éthanol à 80 %, en présence de 1,5 g de triethylamine.

On y ajoute goutte à goutte sous agitation 4,0 g (4,4 ml) de chlorure d'oléoyle.

On agite le mélange 2 heures à la température ambiante puis on y ajoute 50 ml d'eau, suivi de 1 ml d'acide acétique.

On essore le précipité, et on le lave par de l'éther de pétrole et de l'eau.

On recristallise la substance dans l'alcool.

F: 200°C; $\alpha_D = -60°$ (C = 0,5 % éthanol).


## Exemple 5

Préparation de caproyl-L-alanyl-L-alanyl-L-alanine.

On dissout 1,5 g de L-alanyl-L-alanyl-L-alamine dans 20 ml d'éthanol à 75 % contenant 1,56 ml de triéthylamine.

On y ajoute, goutte à goutte, sous agitation à température ambiante, 1,35 ml de chlorure de caproyle en 15 minutes.

On abandonne le mélange 2 heures à température ambiante, puis on y ajoute 30 ml d'eau et 2 ml d'acide acétique.

On abandonne le mélange 2 heures à 0° puis on essore les cristaux et les lave par 2 x 5 ml d'eau glacée et d'éther.

On recristallise la substance dans l'alcool à 70 %.

F: 226°C; $\alpha_D = -43°$ (C = 0,5 % éthanol).


## Exemple 6

Préparation de l'oléoyl-L-alanyl-L-alanyl-L-prolyl-L-valine.

On dissout 0,98 g de L-alanyl-L-alanyl-L-prolyl-L-valine (chlorohydrate) dans 15 ml d'éthanol absolu contenant 1 ml de triéthylanine.

On y ajoute sous agitation, à 4°, 1,26 ml de chlorure d'oléoyle, goutte à goutte pendant 15 minutes, puis on agite encore 4 heures à la température ambiante.

On ajuste le pH à 8,5 par addition de NaOH, et on chasse le solvant sous vide. On triture le résidu par l'éther de prétrole et l'on décante l'éther de pétrole.

On reprend le résidu dans 30 ml d'eau puis on ajuste le pH à 4 par addition d'acide acétique; on extrait l'oléoyl peptide par l'acétate d'éthyle. On chasse le solvant sous vide, et on cristallise le produit dans un mélange d'acétate d'éthyle-éther de pétrole.

F: 70°C(déliquescemt).

$\alpha_D = -72°$ (C = 0,5 % éthanol).

De façon analogue on a préparé les dérivés de formule I suivants:

F° $\alpha_D$*

6

Lauroyl - L-Alanyl- L-Alanyl- L-Alanine 219 -86
Oléoyl - L-Alanyl-L-Alanyl- L-Alanine 200 (déc) -60
Stéaroyl- L-Alanyl- L-Alanyl- L-Alanine, 212 -80
Oléoyl - L-Alanyl- L-Alanyl- L-Prolyl- L-Valinol -70
Oléoyl - L-Alanyl- L-Alanyl- L-Prolyl- L-Valinal -68
* C = 0,5 %, éthanol.

Etude des propriétés des composés de formule I.

1. Expériences in vitro

1.a. Interaction de ces composés avec l'élastine soluble.

L'élastine soluble a été purifiée à partir de ligament large de boeuf adulte par la méthode utilisant la soude 0.1N à ébullition. Préparation of insoluble and soluble elastins. ROBERT L. and HORNEBECK W. dans "The Methodology of Connective Tissue Research". Ed. D.A. Hall (Joynson - Bruvvers Ltd, Oxford). pp. 81-104 (1976). Afin d'étudier l'adsorption des dérivés oléoylés sur ces polymères, les dérivés oléoylés-$(Ala)_2$-Pro-Ala et oléoyl-$(Ala)_2$-Pro-Val ont été synthétisées à l'aide d'acide oléique radiomarqué.

01* -$(Ala)_2$ Pro Ala Activité spécifique: 2.3 $10^3$ cpm/nanomole
01* -$(Ala)_2$ Pro Val Activité spécifique: 2.2 $10^3$ cpm/nanomole
Remarque: "01" est une abréviation pour: oléoyl.

L'élastine (à différentes concentrations) et ces composés radioactifs (à différentes concentrations), sont incubés pendant 24 heures à 37°C dans 1ml d'une solution tampon (100mM tris HCl, $CaCl_2$ 5mM, $NaN_3$ 0,02% pH 8,0).

Les tubes sont centrifugés à 10.000g et le résidu hydrolysé par de la potasse 1 M en présence de 80% éthanol. La radioactivité contenue dans ces hydrolysats est quantifiée et des résultats exprimés en mmole de substance absorbée par mg d'élastine.

Les résultats obtenus montrent que ces composés se fixent sur l'élastine.

1.b. Ces substances et notamment les dérivés alaninal agissent comme inhibiteurs des élastases.

Elastases purifiées utilisées: élastase pancréatique de porc (120 U/mg) et élastase leucocytaire humaine purifiée à partir de la rate.

Concentration molaire de ces enzymes: Elastase pancréatique: $4.10^{-9}M$

Elastase leucocytaire: $10.10^9M$

L'activité enzymatique de ces élastases est suivie par l'hydrolyse de substrats synthétiques spécifiques: N Succionoyltrialanine paranitroanilide, Acétyl-bis alanyl-Propyl-alanine paranitroanilide à une concentration finale de 1,25mM dans un tampon Tris/HC1 pH 8 - 8,6. La variation de densité optique à 410nm est suivie directement dans un spectrophotomètre Beckman type Acta C III.

Dans les expériences d'inhibition, les composés oléyles sont préincubés 15 minutes avec les élastases avant de déterminer les activités enzymatiques résiduelles.

Les résultats sont résumés dans le tableau 1.

**Tableau 1**

| COMPOSE ETUDIE (inhibiteur) | ENZYME | RAPPORT MOLAIRE inh/enz. | POURCENTAGE D'INHIBITION |
|---|---|---|---|
| Ol(Ala)$_2$ Pro Alanine | Elastase pancréatique | $4.10^4$ | 15% |
| Ol(Ala)$_2$ Pro Alaninal | "    " | $4.10^4$ | 85% |
| Ol(Ala)$_2$ Pro Alanine | Elastase leucocytaire | $4.10^4$ | 5% |
| Ol(Ala)$_2$ Pro Valine | " | $4.10^4$ | 51% |
| Ol(Ala)$_2$ Pro Alaninal | " | $4.10^4$ | 90% |

1.c. L'élastine prétraitée avec ces substances est partiellement réfractaire à l'hydrolyse enzymatique par les élastases.

Pour ces expériences, l'elastine a été radiomarquée au borohydrure tritiè Na B$^3$H$_4$, Act sp. 1.75 10$^5$ cpm/mg.

1mg d'èlastine est traité par 10ml d'une solution des différents composés dans 1ml de tampon Tris HCl pH 8,0 durant 24 heures; le mélange est centrifugé et l'élastine insoluble lavée avec 1ml de tampon 0.05mg d'élastase pancréatique sont ensuite ajoutés et l'hydrolyse du polymère est quantifiée par la mesure des peptides radioactifs libérés au cours de l'hydrolyse.

Les résultats obtenus sont résumés dans le tableau 2.

**Tableau 2**

| COMPOSE ETUDIE | RAPPORT MOLAIRE INH./ENZ. | % D'INHIBITION PAR RAPPORT AUX TEMOINS SANS PRETRAITEMENT |
|---|---|---|
| H(Ala)$_2$ Pro Valine | $5.10^4$ | 0 |
| H(Ala)$_2$ Pro Alanine | $5.10^4$ | 0 |
| Ol(Ala)$_2$ Pro Valine | $5.10^4$ | 46% |
| Ol(Ala)$_2$ Pro Alaninal | $5.10^4$ | 98% |

2. <u>Etude in vivo de l'action inhibitrice de l'Oleoyl-bis alanyl-Proline-Alanine envers la dégradation enzymatique des fibres élastinges cutanées induite par injection intradermique d'élastase pancréatique.</u>

Pour ces expérimentations, de jeunes lapins de Garenne (1 mois) ont été utilisés, 3 types d'injections intradermiques (volume:0.25ml; solvant: Tampon phosphate pH 7,0) ont été pratiquées dans le dos des animaux d'expérience.

1. Injections témoins: Injection de 0.25m1 de tampon phosphate seul

2. Injections d'élastase pancréatique de porc: 10 microgrammes (1.2 unités 4.10^{10} mole) dans 0.25ml de tampon.

3. Injections de 50-250 microgrammes d'Ol-(Ala)$_2$-Pro-Ala (8.10^{-8} - 3.10^{-7} mole) dans 0,25ml de tampon suivi d'une injection au même site de 10 microgrammes (1.2 unités, 4.10^{10} mole) d'élastase pancréatique dans 0,25ml de tampon.

Les différents échantillons de peau de lapin sont ensuite traités en histologie afin de permettre la visualisation des fibres élastiques.

L'observation histologique et des études de morphométrie quantitative font apparaitre:
- que 40-60% du tissu élastique sont préservés dans le cas du prétraitement avec le dérivé oléoylé (Rapport molaire inhibiteur/enzyme = 2.10$^2$).
- que 60-80% du tissu élastique sont préservés dans le cas du prétraitement du tissu avec le dérivé oléoylé (Rapport molaire inhibiteur/enzyme = 10$^3$).

**Conclusions:**

a) Les substances étudiés:
01-(Ala)$_2$-Pro-Ala,
01-(Ala)$_2$-Pro-Val,
01-(Ala)-Pro-Ala-CHO
agissent comme inhibiteurs des élastases;

b) Ces composés se lient à l'élastine, et sous forme absorbée aux fibres élastiques, sont capables de diminuer considérablement l'action élastolytique des élastases, et cela aussi bien in vivo qu'in vitro.

**Revendications**

1. Lipopeptides de formule générale:
R-X-(P$_1$)$_x$-(L-Ala-L-Ala-P$_2$)-A (I) dans laquelle:
x représente le nombre 0 ou 1;
R représente le reste acyle d'un acide carboxylique hydrophobe tel qu'un acide carboxylique aliphatique ayant 6 à 25 atomes de carbone et pouvant renfermer éventuellement 1 à 5 doubles liaisons, d'un acide carboxylique alicyclique ayant 6 à 25 atomes de carbone, d'un acide arylcarboxylique ou d'un acide carboxylique arylaliphatique dont le groupement aryle comprend 1 à 2 cycles et dont le groupement aliphatique comprend 1 à 18 atomes de carbone, les groupements aromatiques desdits groupements aryle ou arylaliphatique étant éventuellement substitués;

P$_2$ représente un résidu d'acide aminé ou de dipeptide, relié par son extrémité N-terminale au groupement L-Ala adjacent, choisi dans le groupe constitué par les acides aminés et dipeptides suivants:
-L-Ala-, -L-Val-, -Gly-, -L-Mét-, -L-Pro-, -L-Leu-, -L-Pro-L-Val-, -L-Pro-L-Ala-, -L-Pro-L-Phé-, -L-Pro-L-Leu, -L-Pro-L-Mét- et -L-Pro-Gly-;

P$_1$ représente un résidu d'acide aminé ou d'oligopeptide formé de 2 à 8 aninoacides de la série L, lesdits aminoacides étant choisis dans le groupe constitué par la glycine, l'alanine, la valine, la méthionine, la leucine, l'isoleucine, la proline, la phénylalanine, la sérine, la cystéine, la cystine, l'arginine, la tyrosine, l'ornithine, la lysine et l'acide glutamique,

étant entendu que P$_1$ est relié à RX- par son groupement N-terminal et que P$_2$ est relié à A par son groupement C-terminal,

Ala est la représentation conventionnelle de l'alanine,

X represente une liaison covalente directe reliant R soit au groupement N-terminal (-NH-) du premier aminoacide de P$_1$ (cas où x=1) soit au groupement N-terminal (-NH-) du premier groupement Ala représenté à gauche sur la formule I (cas où x = 0),

ou bien X est un groupement divalent ayant 2 à 10 atomes de carbone jouant le rôle de "bras" entre le groupement R et le reste de la molécule de formule I,

et A représente la partie C-terminale du peptide -(P$_1$)$_x$-(Ala-Ala-P$_2$) -A, A étant choisi parmi un groupement carboxylique -CO$_2$H ou ses dérivés (sels, esters), -CHO, -CONH$_2$, -COCH$_2$Cl et -CH$_2$OH.

2. Lipopeptides selon la revendication 1, caractérisés par le fait que R représente le reste acyle d'un acide gras ayant 6 à 20 atomes de carbone, tel que l'acide laurique ou l'acide oléique, le reste de l'acide chénodéoxycholique ou de l'acide cholique, ou le reste acyle d'un acide phénylalcanoïque éventuellement substitué sur le noyau benzénique.

3. Lipopeptides selon l'une quelconque des revendications précédentes caractérisés par le fait que X est un groupement -Z-(CH$_2$)$_n$-CO-, Z représentant -O- ou -NH-, n'étant un nombre entier variant de 5 à 20.

4. Lipopeptides selon l'une quelconque des revendications précédentes caractérisés par le fait que l'oligopeptide que représente -(P$_1$)$_x$-(L-Ala-L-Ala-P$_2$) - ne possède pas plus de 10 aminoacides.

5. Lipopeptides selon l'une quelconque des revendications précédentes, caractérisés par le fait que A représente un groupement -CO-OY, Y étant un groupement aliphatique, aryle ou arylaliphatique éventuellement substitué.

6. Lipopeptides selon la revendication 1, caractérisés par le fait qu'ils sont choisis parmi les suivants:
- oléoyl-L-Alanyl-L-Alanyl-L-Prolyl-L-Alanine;
- lauroyl-trialanine;
- Oléoyl-Alanyl-Alanyl-Proline;
- Oléoyl-Alanyl-Alanyl-Prolyl-Alaninol;
- Oléoyl-Alanyl-Alanyl-Prolyl-Alaninal;
- Caproyl - L-Alanyl- L-Alanyl- L-Alanine;
- Lauroyl - L-Alanyl- L-Alanyl- L-Alanine;
- Oleoyl - L-Alanyl- L-Alanyl- L-Alanine;
- Stearoyl- L-Alanyl- L-Alanyl- L-Alanine;
- Oleoyl - L-Alanyl- L-Alanyl- L-Proline;
- Oleoyl - L-Alanyl- L-Alanyl- L-Prolyl- L-Alanine;
- Oleoyl - L-Alanyl- L-Alanyl- L-Prolyl- L-Valine;
- Oleoyl - L-Alanyl- L-Alanyl- L-Prolyl- L-Alaninol
- Oleoyl - L-Alanyl- L-Alanyl- L-Prolyl- L-Valinol;
- Oleoyl - L-Alanyl- L-Alanyl- L-Prolyl- L-Alaninol;
- Oleoyl - L-Alanyl- L-Alanyl- L-Prolyl- L-Valinal.

7. Procédé de préparation des lipopeptides selon l'une quelconque des revendications précédentes, caractérisé par le fait que l'on utilise comme produit de départ un composé de formule génerale II

H-X$_1$-(-P$_1$-) -(L-Ala-L-Ala-P$_3$-) -A$_1$ (II) dans laquelle X$_1$ a la même définition que X ou bien représente une liaison covalente directe entre H- et -P$_1$- ;

P$_3$ i la même définition que P$_2$, ou P$_3$ représente un groupement -L-Pro-, ou bien P$_3$ représente une liaison covalente directe entre A$_1$ et le groupement -L-Ala- immédiatement adjacent;

A$_1$ représente un groupement - CO$_2$H, - CO-OY (Y étant défini comme précédemment), - CHO ou - CONH$_2$;

et x est défini comme précédemment;

que l'on fait réagir ledit produit de départ, éventuellement présent sous la forme d'un sel d'addition comme le chlorhydrate, avec un réactif de formule III

R-X$_2$-Z$_1$ (III)

dans laquelle R est défini comme précédemment;

X$_2$ a la même définition que X lorsque X$_1$ représente une liaison covalente, et X$_2$ représente une liaison covalente directe entre R et Z$_1$ lorsque X$_1$ a la même définition que X; et Z$_1$ est un groupement réactif permettant la réaction de R-X$_2$-Z$_1$ sur le composé II avec élimination d'un composé Z$_1$H et formation d'un composé de formule IV

R-X-(P$_1$)$^x$-(L-Ala-L-Ala-P$_3$-) -A$_1$ (IV);

que, dans le cas où P$_3$ représente le groupement -L-Pro-, on fait réagir le composé de formule IV avec un acide aminé de la série L choisi parmi la valine, l'alanine, la phénylalanine, la leucine, la méthionine et la glycine, ou avec un dérivé d'un desdits acides aminés, pour obtenir un dérivé de formule I; puis que l'on transforme, si désiré, le composé obtenu en tout autre composé de formule I selon les méthodes connues, en particulier en remplaçant le groupement terminal A ou A$_1$ en tout autre groupement terminal répondant à la définition de A donnée ci-dessus.

8. Utilisation des lipopeptides tels que définis dans l'une quelconque des revendications 1 à 6 comme inhibiteurs des élastases et/ou comme protecteurs de la fibre élastique.

9. Utilisation selon la revendication 8, caractérisée par le fait que l'on utilise lesdits lipopeptides comme inhibiteurs de l'élastase de la peau et comme protecteurs de la peau.

10. Utilisation selon la revendication 9, caractérisée par le fait que l'on applique lesdits lipopeptides sur la peau en vue de conserver ou de restaurer la souplesse de la peau et de prevenir ou de retarder la formation de rides.

11. Compositions inhibitrices des protéases du type élastase et/ou protectrices de la fibre élastique, caractérisées par le fait qu'elles contiennent au moins un lipopeptide tel que défini dans l'une quelconque des revendications 1 à 6, en mélange avec un excipient approprié.

12. Compositions selon la revendication 11, caractérisées par le fait qu'elles constituent une composition pharmaceutique contenant ledit lipopeptide comme ingrédient actif, ladite composition étant utilisable notamment à titre de traitement principal ou complémentaire de l'artériosclérose, de l'emphysème pulmonaire, de l'arthrose, du diabéte et de certaines tumeurs dans lesquelles les élastases sont impliquées.

13. Composition selon la revendication 11, caractérisé par le fait qu'elles constituent une composition cosmétique contenant comme ingrédient actif au moins un desdits lipopeptides, en combinaison avec un excipient habituellement utilisé dans les compositions cosmétiques pour la peau.

## Claims

1. Lipopeptides of general formula:
R-X-(P1)$_x$-(L-Ala-L-Ala-P$_2$) -A (I)
in which:
x denotes the number 0 or
R denotes the acyl residue of a hydrophobic carboxylic acid, such as an aliphatic carboxylic acid which has 6 to 25 carbon atoms and can optionally contain 1 to 5 double bonds, of an alicyclic carboxylic acid having 6 to 25 carbon atoms, of an arylcarboxylic acid or of an arylaliphatic carboxylic acid in which the aryl group comprises 1 or 2 rings and in which the aliphatic group comprises 1 to 18 carbon atoms, the aromatic groups of the said aryl or arylaliphatic groups optionally being substituted;

P$_2$ denotes an amino acid residue or dipeptide residue, connected through its N-terminal end to the adjacent L-Ala group, chosen from the group consisting of the following amino acids and dipeptides:
-L-Ala-, -L-Val-, -Gly-, -L-Met-, -L-Pro-, -L-Leu-, -L-Pro-L-Val-, -L-Pro-L-Ala-, -L-Pro-L-Phe-, -L-Pro-L-Leu, -L-Pro-L-Met- and -L-Pro-Gly-;

P$_1$ denotes an amino acid residue or oligopeptide residue consisting of 2 to 8 amino acids of the L- series, the said amino acids being chosen from the group consisting of glycine, alanine, valine, methionine, leucine, isoleucine, proline, phenylalanine, serine, cysteine, cystine, arginine, tyrosine, ornithine, lysine and glutamic acid,

with the proviso that P$_1$ is connected to RX-through its N-terminal group and that P$_2$ is connected to A through its C-terminal group,

Ala is the conventional representation of alanine,

X denotes a direct covalent bond connecting R either to the N-terminal group (-NH-) of the first amino acid of P$_1$ (in the case where x = 1), or to the N-terminal group (-NH-) of the first Ala group shown at the left in the formula I (in the case where x = 0),

or alternatively X is a divalent group having 2 to 10 carbon atoms, performing the role of "arm" between the group R and the remainder of the molecule of formula I,

and A denotes the C-terminal portion of the peptide -(P$_1$)$_x$-(Ala-Ala-P2) -A, A being chosen from a carboxylic group -CO2H or the derivatives thereof (salts, esters), -CHO, -CONH$_2$, -COCH$_2$Cl and -CH$_2$OH.

2. Lipopeptides according to Claim 1, characterized in that R denotes the acyl residue of a fatty acid having 6 to 20 carbon atoms, such as lauric acid or oleic acid, a chenodeoxycholic acid or cholic acid residue, or the acyl residue of a phenylalkanoic acid optionally substituted on the benzene ring.

3. Lipopeptides according to any one of the preceding claims, characterized in that X is a group -Z-(CH$_2$)$_n$-CO-, Z denoting -O- or -NH-, n being an integer varying from 5 to 20.

4. Lipopeptides according to any one of the preceding claims, characterized in that the oligopeptide represented by -(P1)$_x$-(L-Ala-L-Ala-P2) does not possess more than 10 amino acids.

5. Lipopeptides according to any one of the preceding claims characterized in that A denotes a group -CO-OY, Y being an optionally substituted arylaliphatic, aliphatic or aryl group.

6. Lipopeptides according to Claim 1, characterized in that they are chosen from the following:
- Oleoyl-L-Alanyl-L-Alanyl-L-Prolyl-L-Alanine;
- Lauroyl-trialanine;
- Oleoyl-Alanyl-Alanyl-Proline,
- Oleoyl-Alanyl-Alanyl-Prolyl-Alaninol;
- Oleoyl-Alanyl-Alanyl-Prolyl-Alaninal;
- Caproyl - L-Alanyl- L-Alanyl- L-Alanine;
- Lauroyl - L-Alanyl- L-Alanyl- L-Alanine;
- Oleoyl - L-Alanyl- L-Alanyl- L-Alanine;
- Stearoyl- L-Alanyl- L-Alanyl- L-Alanine;
- Oleoyl - L-Alanyl- L-Alanyl- L-Proline;
- Oleoyl - L-Alanyl- L-Alanyl- L-Prolyl- L-Alanine;
- Oleoyl - L-Alanyl- L-Alanyl- L-Prolyl- L-Valine;
- Oleoyl - L-Alanyl- L-alanyl- L-Prolyl- L-Alaninol;
- Oleoyl - L-Alanyl- L-Alanyl- L-Prolyl- L-Valinol;
- Oleoyl - L-Alanyl- L-Alanyl- L-Prolyl- L-Alaninol;
- Oleoyl - L-Alanyl- L-Alanyl- L-Prolyl- L-Valinal.

7. Process for preparing the lipopeptides according to any one of the preceding claims, characterized in that there is used as the starting substance a compound of general formula II
H-X$_1$-(-P$_1$-)$_x$-(L-Ala-L-Ala-P$_3$-) -A$_1$ (II) in which X$_1$ has the same definition as X or alternatively denotes a direct covalent bond between H- and -P$_1$-;

P3 has the same definition as P$_2$, or P$_3$ denotes an -L-Pro- group, or alternatively P$_3$ denotes a direct covalent bond between Al and the immediately adjacent -L-Ala- group;

A$_1$ denotes a group -CO2H, -CO-OY (Y being as defined above), -CHO or -CONH$_2$,.

and X is defined as above;

in that the said starting substance, optionally present in the form of an addition salt such as the hydrochloride, is reacted with a reagent of formula III

R-X$_2$-Z$_1$ (III)

in which R is defined as above;

X$_2$ has the same definition as X when X$_1$ denotes a covalent bond, and X$_2$ denotes a direct covalent bond between R and Z$_1$ when X$_1$ has the same definition as X; and Z$_1$ is a reactive group which enables R-X$_2$-Z$_1$ to be reacted with the compound II, with elimination of a compound Z$_1$H and formation of a compound of formula IV;

R-X-(P$_1$)$_x$-(L-Ala-L-Ala-P$_3$-) -A$_1$ (IV),

in that, in the case where P$_3$ denotes a -L-Progroup, the compound of formula IV is reacted with an amino acid of the L- series chosen from valine, alanine, phenylalanine, leucine or methionine and glycine, or with a derivative of one of the said amino acids, to obtain a derivative of formula I; and in that, if desired, the compound obtained is then converted to any other compound of formula I according to known methods, especially by replacing the terminal A or A$_1$ group with any other terminal group corresponding to the definition of A given above.

8. Use of the lipopeptides as defined in any of Claims 1 to 6 as elastase inhibitors and/or as protective agents for elastic fibres.

9. Use according to Claim 8, characterized in that the said lipopeptides are used as inhibitors of elastase in the skin and as protective agents for the skin.

10. Use according to Claim 9, characterized in that the said lipopeptides are applied on the skin for the purpose of preserving or restoring the suppleness of the skin and preventing or delaying the formation of wrinkles.

11. Compositions which are inhibitory to proteases of the elastase type and/or are protective of elastic fibres, characterized in that they contain at least one lipopeptide as defined in any one of Claims 1 to 6, mixed with a suitable excipient.

12. Compositions according to Claim 11, characterized in that they constitute a pharmaceutical composition containing the said lipopeptide as active ingredient, the said composition being usable, in particular, by way of a main or supplementary treatment of arteriosclerosis, pulmonary emphysema, osteoarthritis, diabetes and certain tumours in which elastases are implicated.

13. Compositions according to Claim 11, characterized in that they constitute a cosmetic composition containing as active ingredient at least one of the said lipopeptides, in combination with an excipient customarily used in cosmetic compositions for the skin.


## Patentansprüche

1. Lipopeptide der allgemeinen Formel:

R-X-(P$_1$)$_x$-(L-Ala-L-Ala-P$_2$) -A (I)

in der

x die Zahl null oder 1 darstellt,

R den Acylrest einer hydrophoben Carbonsäure, wie einer aliphatischen Carbonsäure mit 6 bis 25 Kohlenstoffatomen, die gegebenenfalls 1 bis 5 Doppelbindungen enthalten kann, einer alicyclischen Carbonsäure mit 6 bis 25 Kohlenstoffatomen, einer Arylcarbonsäure oder einer arylaliphatischen Carbonsäure, deren Arylgruppe 1 bis 2 Ringe und deren aliphatische Gruppe 1 bis 18 Kohlenstoffatome enthält, wobei die Gruppen gegebenenfalls substituiert sind, darstellt;

P$_2$ einen Aminosäurerest oder ein Dipeptid darstellt, der bzw. das durch das äußerste Ende des endständigen N-Atoms der benachbarten L-Ala-Gruppe gebunden ist, ausgewählt aus der Gruppe der folgenden Aminosäuren und Dipeptide:

-L-Ala-, -L-Val-, -Gly-, -L-Met-, -L-Pro-, -L-Leu-, -L-Pro-L-Val-,

-L-Pro-L-Ala-, -L-Pro-L-Phe-, -L-Pro-L-Leu, -L-Pro-L-Met- und -L-Pro-Gly-;

P$_1$ einen Rest einer Aminosäure oder des aus 2 bis 8 Aminosäuren der L-Reihe gebildeten Oligopeptids darstellt, wobei diese Aminosäuren ausgewählt sind aus der Gruppe bestehend aus Glycin, Alanin, Valin, Methionin, Leucin, Isoleucin, Prolin, Phenylalanin, Serin, Cystein, Cystin, Arginin, Tyrosin, Ornithin, Lysin und der Glutaminsäure, wobei P$_1$ an RX über seine endständige N-Gruppe und P$_2$ an A über seine endständige C-Gruppe gebunden ist, Ala die übliche Wiedergabe von Alanin ist,

X eine kovalente direkte Bindung ist, die R entweder an die endständige N-Gruppierung (-NH-) der ersten Aminosäure von P$_1$ (im Fall von x=l) oder an die endständige N-Gruppierung (-NH-) der ersten Gruppe Ala, die in Formel I links steht (im Fall von x=O) bindet,

oder aber X eine zweiwertige Gruppe mit 2 bis 10 Kohlenstoffatomen ist, welche die Rolle des "Arms" zwischen der Gruppe R und dem Rest des Moleküls der Formel I spielt und A den Teil des endständigen C des Peptids -(P$_1$)$_x$-(Ala-Ala-P$_2$) -A

darstellt, wobei A ausgewählt ist aus einer Carboxylgruppe -CO$_2$H oder deren Derivate (Salze, Ester) oder Gruppen -CHO, -CONH$_2$, COCH$_2$Cl und -CH$_2$OH.

2. Lipopeptide nach Anspruch 1, dadurch gekennzeichnet, daß R den Acylrest einer Fettsäure mit 6 bis 20 Kohlenstoffatomen, wie der Laurinsäure oder ölsäure, der Rest der Chenodesoxycholsäure oder der Cholsäure oder der Acylrest einer Phenylalkancarbonsäure, der gegebenenfalls durch einen Benzolkern substituiert ist, bedeutet.

3. Lipopeptide nach den vorhergehenden Ansprüchen, dadurch gekennzeichnet, daß wenn X eine Gruppe -Z-$(CH_2)_n$-CO- ist, Z -O- oder -NH- darstellt und n eine ganze Zahl von 5 bis 20 ist.

4. Lipopeptide nach den vorhergehenden Ansprüchen, dadurch gekennzeichnet, daß das Oligopeptid, das -$(P_1)_x$-(L-Ala-L-Ala-$P_2$) darstellt, nicht mehr als 10 Aminosäuren aufweist.

5. Lipopeptide nach den vorhergehenden Ansprüchen, dadurch ge- kennzeichnet, daß wenn A eine Gruppe -CO-OY ist, Y eine aliphatische Gruppe, Arylgruppe oder arylaliphatische Gruppe, die gegebenenfalls substituiert sind, bedeutet.

6. Lipopeptide nach Anspruch 1, dadurch gekennzeichnet, daß sie aus folgenden ausgewählt sind:

Oleoyl-L-Alanyl-L-Alanyl-L-Propyl-L-Alanin;

Lauroyl-trialanin;

Oleoyl-Alanyl-Alanyl-Prolin;

Oleoyl- Alanyl-Alanyl-Prolyl-Alaninol;

Oleolyl-Alanyl-Alanyl-Prolyl-Alaninal;

Caproyl - L-Alanyl- L-Alanyl- L-Alanin;

Lauroyl - L-Alanyl- L-Alanyl- L-Alanin;

Oleoyl - L-Alanyl- L-Alanyl- L-Alanyl- L-Alanin;

Stearoyl- L-Alanyl- L-Alanyl- L-Alanin;

Oleoyl -L-Alanyl- L-Alanyl- L-Prolin;

Oleoyl -L-Alanyl- L-Analyl- L-Prolyl- L-Alanin;

Oleoyl -L-Alanyl- L-Alanyl- L-Prolyl- L-Valin;

Oleoyl -L-Alanyl- L-Alanyl- L-Prolyl- L-Alaninol;

Oleoyl -L-Alanyl- L-Alanyl- L-Prolyl- L-Valinol;

Oleoyl -L-Alanyl- L-Alanyl- L-Propyl- L-Alaninol.

Oleoyl -L-Alanyl- L-Alanyl- L-Prolyl- L-Valinal.

7. Verfahren zur Herstellung von Lipopeptiden nach den vorhergehenden Ansprüchen, dadurch gekennzeichnet, daß man als Ausgangsprodukt eine Verbindung der allgemeinen Formel II verwendet:

H-$X_1$-$(P_1$-$)_x$-(L-Ala-L-Ala-$P_3$) -$A_1$ (II)

in der $X_1$ die gleiche Bedeutung wie X hat oder eine direkte kovalente Bindung zwischen H- und -$P_1$- bedeutet $P_3$ die gleiche Bedeutung wir $P_2$ hat oder $P_3$ eine L-Pro-Gruppe darstellt oder $P_3$ eine direkte kovalente Bindung zwischen $A_1$ und der Gruppe -L-Ala- darstellt, welche unmittelbar angrenzt;

$A_1$ eine Gruppierung - $CO_2H$, - CO-OY (wobei Y die oben genannte Bedeutung hat), - CHO oder - $CONH_2$ darstellt, und x wie oben definiert ist, daß man das Ausgangsprodukt, das gegebenenfalls in Form eines Additionssalzes, wie das Hydrochlorid, vorliegt, mit einem Reaktionsteilnehmer der Formel III umsetzt

R-$X_2$-$Z_1$ (III)

in der R die oben genannte Bedeutung hat;

$X_2$ die gleiche Bedeutung wie X hat oder $X_1$ eine kovalente Bindung und $X_2$ eine direkte kovalente Bindung zwischen R und $Z_1$ darstellt, oder $X_1$ die gleiche Bedeutung wie X hat; und wobei $Z_1$ eine reaktive Gruppe ist, die die Reaktion von R-$X_2$-$Z_1$ mit der Verbindung II unter Eliminierung der Verbindung $Z_1$H und Bildung einer Verbindung der Formel IV gestattet:

R-X-$(P_1)_x$-(L-Ala-L-Ala-$P_3$) -$A_1$ (IV)

und daß man in dem Fall, in dem $P_3$ die Gruppe -L-Pro- darstellt, die Verbindung der Formel IV mit einer Aminosäure der L-Reihe ausgewählt aus Valin, Alanin, Phenylalanin, Leucin, Methionin und Glycin oder mit einem Derivat dieser Aminosäuren umsetzt, um ein Derivat der Formel I zu erhalten; daß man gegebenenfalls die erhaltene Verbindung in irgendeine andere Verbindung, der Formel I nach bekannten Verfahren überführt, insbesondere durch Ersetzen der Endgruppe A oder $A_1$ in irgendeine andere Endgruppe, die der oben angegebenen Bedeutung von A entspricht.

8. Verwendung von Lipopeptiden, wie sie in einem oder mehreren Ansprüchen 1 bis 6 definiert sind, als Elastase-Inhibitoren und/oder als Schutzsubstanzen für-elastische Fasern.

9. Verwendung nach Anspruch 8, dadurch gekennzeichnet, daß man die Lipopeptide als Inhibitoren von Elastase der Haut und als Hautschutzmittel verwendet.

10. Verwendung nach Anspruch 9, dadurch gekennzeichnet, daß man die Lipopeptide auf der Haut aufbringt, um die Geschmeidigkeit der Haut zu erhalten oder zurückzugewinnen und der Bildung von Falten zuvorzukommen oder diese zu verzögern.

11. Inhibierungsmittel für Proteasen vom Typ Elastase und/oder Schutzmittel für elastische Fasern, dadurch gekennzeichnet, daß mindestens ein Lipopeptid wie in einem oder mehreren der Ansprüche 1 bis 6 definiert sind, im Gemisch mit einem geeigneten Exzipienten enthalten.

12. Mittel nach Anspruch 11, dadurch gekennzeichnet, daß sie aus einer pharmazeutischen Zubereitung bestehen, die das Lipopeptid als aktiven Bestandteil enthält, wobei diese Zubereitung insbesondere zur hauptsächlichen oder ergänzenden Behandlung von Arteriosklerose, Lungenemphysem, Arthrose Diabetes und bestimmten Tumoren, bei denen Elastasen eine Rolle spielen, verwendbar ist.

13. Mittel nach Anspruch 11, dadurch gekennzeichnet, daß sie aus einer kosmetischen Zubereitung bestehen, die als aktiven Bestandteil mindestens eines dieser Lipopeptide enthalten, in Kombination mit einem Exzipienten, wie er üblicherweise in kosmetischen Zubereitungen für die Haut verwendet wird.